# EUROPEAN PATENT SPECIFICATION

(11) **EP 3 345 557 B1**
(45) Date of publication and mention of the grant of the patent: **06.04.2022**
(21) Application number: 18150803.7
(22) Date of filing: 09.01.2018
(51) Int. Cl.: A61B 17/32, A61B 17/24, A61B 18/14, A61B 18/00, A61B 17/00

(54) **COMBINED DEBRIDER AND COAGULATOR**
KOMBINIERTER DEBRIDER UND KOAGULATOR
DÉBRIDEUR ET COAGULATEUR COMBINÉS

(30) Priority: 10.01.2017 US 201715403060
(43) Date of publication of application: 11.07.2018
(73) Proprietor: Biosense Webster (Israel) Ltd., Yokneam 2066717 (IL)
(72) Inventor: GOVARI, Assaf, 2066717 Yokneam (IL); ALGAWI, Yehuda, 2066717 Yokneam (IL); SITNITSKY, Ilya, 2066717 Yokneam (IL)
(74) Representative: Carpmaels & Ransford LLP

(56) References cited:
- WO-A1-2015/175176
- WO-A1-2016/018457
- WO-A2-2005/009213
- WO-A2-2005/023084
- US-A1- 2014 100 567
- US-B1- 6 428 539
- US-B1- 7 150 747

## Description

### FIELD OF THE INVENTION

The present invention relates generally to invasive medical probes, and specifically to an invasive medical probe configured to perform therapies within the nasal cavity and the paranasal sinuses.

### BACKGROUND OF THE INVENTION

Functional endoscopic sinus surgery (FESS) is a common type of surgery used to treat chronic sinusitis. In a typical FESS procedure, anatomical structures are typically removed or modified in order to access the openings into the paranasal sinus. For example, during a FESS procedure, one or more rigid surgical instruments (e.g., a rigid endoscope) can be inserted into a nostril in order to perform operations such as removing bone and mucosal tissue from the nasal cavity and the openings into the paranasal sinuses, and enlarging such openings to improve sinus drainage and to mitigate sinusitis symptoms. Other types of tissue that can be treated during FESS surgery include polyps and bony growths.

US7150747B1 describes an electrosurgical cutter. US2014/100567A1 describes a surgical cutting instrument with electromechanical cutting. WO2016/018457A1 describes a surgical endoscopic cutting system. US6428539B1 describes an apparatus and method for minimally invasive surgery using rotational cutting tool. WO2005/009213A2 describes a rotary electrosurgical apparatus. WO2005/023084A2 describes a surgical instrument and methods of using for otolaryngology surgeries.

The description above is presented as a general overview of related art in this field and should not be construed as an admission that any of the information it contains constitutes prior art against the present patent application.

### SUMMARY OF THE INVENTION

The invention is defined by the appended claims. There is provided, in accordance with an embodiment of the present invention, a medical apparatus, including an insertion tube including an outer tube having a proximal end and a distal end configured for insertion into a body cavity, with a radial cutting window opening through a wall of the outer tube in proximity to the distal end, and an inner tube, contained in and configured to rotate within the outer tube and including a cutting blade that traverses the radial cutting window opening as the inner tube rotates. The medical apparatus also includes a conductive element disposed over an outer surface of the distal end of the insertion tube, and a conductor running along the insertion tube from the proximal to the distal end and coupled to provide energy to the conductive element. The conductive element is configured to inject energy into the body cavity in order to perform coagulation. The distal end includes an outer tube distal end, wherein the inner tube has an inner tube distal end and an inner tube proximal end, and including a radial suction window opening through a wall of the inner tube in proximity to the inner tube distal end, the radial suction window opening being configured to traverse the radial cutting window opening at specific angles of rotation, and wherein the inner tube proximal end is coupled to a suction device configured to extract debris via the radial suction window opening. The medical apparatus is configured to restrict conveyance of energy to the conductive elements to times when the radial cutting window opening and the radial suction window opening are aligned.

In some embodiments, the outer surface of the distal end includes the outer surface of the outer tube. In additional embodiments, the outer surface of the distal end includes the outer surface of the inner tube. In embodiments where the outer surface of the distal end includes the outer surface of the inner tube, the conductor can be configured to provide the energy to the conductive element when the cutting blade closes the radial cutting window.

In further embodiments, the cutting blade includes a first cutting blade, and the medical apparatus includes a second cutting blade disposed on an edge of the radial cutting window opening. In some embodiments, the second cutting blade includes a set of grasping teeth configured to grasp body cavity tissue. In supplemental embodiments, the conductive element cam be disposed over the second cutting blade.

In embodiments where the medical apparatus includes a radial suction window opening, the cutting blade may be disposed on an edge of the radial suction window opening.

In further embodiments, the medical apparatus may include input devices that independently control the rotation of the inner tube and the provision of the energy to the conductive element in order to vary a ratio of cutting and heating times. In supplemental embodiments, the body cavity may be selected from a group consisting of a nasal cavity and a paranasal sinus. In some embodiments, the energy includes radio-frequency energy.

There is also provided, in accordance with an embodiment of the present invention, a method according to the appended claims.

### BRIEF DESCRIPTION OF THE DRAWINGS

The disclosure is herein described, by way of example only, with reference to the accompanying drawings, wherein:
Figure 1 is a schematic pictorial illustration of a medical system configured to perform a procedure using a medical probe that combines a debrider and a coagulator, in accordance with an embodiment of the present invention;
Figure 2 is a schematic sectional view of the medical probe, in accordance with an embodiment of the present invention;
Figures 3 and 4 are schematic sectional views of a distal end of the medical probe, in accordance with an embodiment of the present invention; and
Figures 5 and 6 are schematic detail views showing the distal end of the medical probe in a nasal cavity during a medical procedure.

### DETAILED DESCRIPTION OF EMBODIMENTS

### OVERVIEW

Various therapeutic procedures such as functional endoscopic sinus surgery (FESS) typically use one or more invasive medical probes that are inserted into a cavity in a patient's body. Examples of invasive medical probes that are used in FESS procedures include debriders (also known as shavers and cutters) that are configured to cut bone and tissue, and coagulators that are configured to perform coagulation of soft tissue and hemostasis of blood vessels.

Embodiments of the present invention combine the functionality of a debrider and a coagulator into a single medical probe. As described hereinbelow, the medical probe comprises an insertion tube that comprises an outer tube having a proximal end and a distal end configured for insertion into a body cavity, and a radial cutting window opening through a wall of the outer tube in proximity to the distal end. The insertion tube also comprises an inner tube that is contained in the outer tube and configured to rotate within the outer tube. The inner tube comprises a cutting blade that traverses the radial cutting window opening as the inner tube rotates, thereby cutting (i.e., "debriding") any bone and tissue protruding into the radial cutting window opening.

In embodiments of the present invention, the medical probe further comprises a conductive element that is disposed over an outer surface of the distal end of the insertion tube. In some embodiments, the conductive element is disposed over an outer surface of the outer tube, and the medical probe comprises a conductor that runs along the outer tube from the proximal to the distal end and that is coupled to provide energy (e.g., radio-frequency energy) to the conductive element. Upon receiving the energy, the conductive element is configured to inject the energy into a body cavity during a medical procedure in order to coagulate fluid in the body cavity.

The medical probe may also comprise an additional cutting blade disposed along an edge of the radial cutting window opening. In some embodiments, the additional cutting blade may comprise a set of grasping teeth configured to grasp body cavity tissue during the medical procedure. In embodiments where the conductive element is disposed over the outer surface of the outer tube, the conductive element may also be disposed over the additional cutting blade and coupled to the conductor, thereby enabling the additional cutting blade to perform coagulation.

In some embodiments, a distal end of the inner tube may comprise a radial suction window opening that is configured to traverse the radial cutting window opening as the inner tube rotates. When the inner tube is coupled to a suction device (also referred to herein as a suction module) at its proximal end, the medical probe can be configured to extract, via the radial suction window opening, debris (e.g., body cavity tissue and coagulated blood) during the medical procedure.

In further embodiments, the medical probe can be configured to restrict conveyance of the energy to the distal end to times when the inner tube is positioned at specific angles of rotation relative to the outer tube. For example, in embodiments where the conductive element is disposed over the outer tube, the medical probe can be configured to restrict conveyance of the energy to the conductive element to times when the radial cutting window opening and the radial suction window openings are aligned, thereby enabling suction in the inner tube to extract coagulated debris. In additional embodiments, the rotation of the inner tube and the energy conveyed to the conductive element can each be controlled independently, thereby enabling an operator to vary the ratio of debriding and coagulation times.

### SYSTEM DESCRIPTION

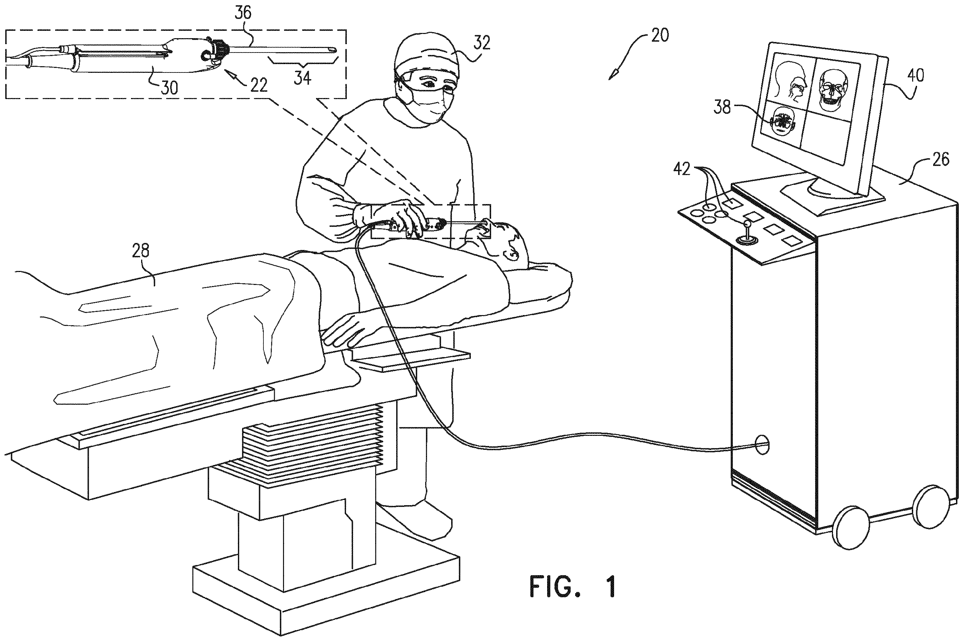

Figure 1 is a schematic pictorial illustration of a medical system 20 comprising a medical probe 22 that combines the functionality of a debrider and a coagulator and a control console 26, in accordance with an embodiment of the present invention. In the example shown in Figure, 1, medical system 20 can be used for minimally invasive catheter-based sinus surgery on a patient 28.

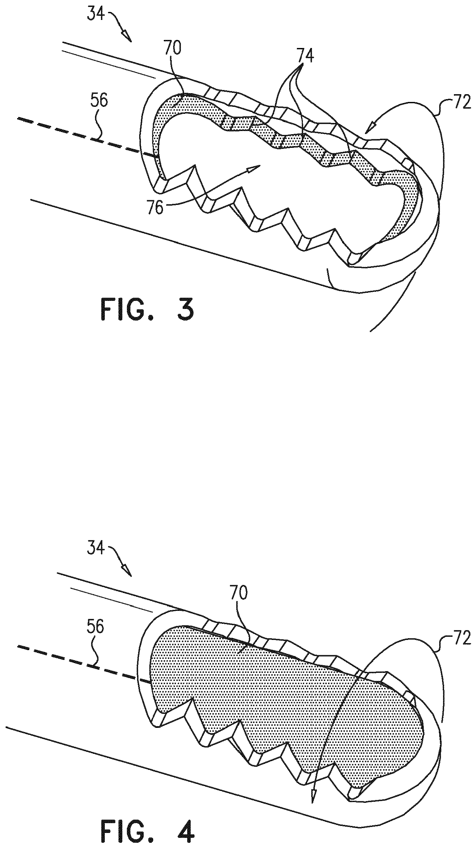

Medical probe 22 comprises a handle 30 that an operator 32 can grasp and manipulate in order to insert a distal end 34 of an insertion tube 35 into a lumen, such as a nasal cavity or a paranasal sinus, of patient 28. In embodiments of the present invention, insertion tube 35 comprises an outer tube 36 and an inner tube that is contained in and configured to rotate within the outer tube. The inner tube is described hereinbelow in the description referencing Figures 3 and 4.

Medical probe 22 typically comprises a position sensor (not shown) that generates a signal indicating a current location of distal end 34 in patient 28. In operation, control console 26 can receive the signal and register the indicated location to a pre-acquired image (e.g., a computerized tomography image) in order to generate a real-time image 38. In some embodiments, control console 26 can generate real-time image 38 by overlaying, on the pre-acquired image, an icon indicating a current registered location of distal end 34. Control console 26 can present the real-time image as information regarding the procedure on a display 40.

Examples of position sensors include magnetic field based position sensors and impedance based position sensors. Magnetic position tracking techniques are described, for example, in U.S. Patents 5,391,199 and 6,690,963 referenced above, and in in U.S. Patents 5,443,489, 6,788,967, 5,558,091, 6,172,499 and 6,177,792. Impedance-based position tracking techniques are described, for example, in U.S. Patents 5,983,126, 6,456,864 and 5,944,022.

Display 40 is assumed, by way of example, to comprise a flat panel display such as a liquid crystal display, a light emitting diode display, an organic light-emitting diode display or a plasma display. However, other display devices can also be employed to implement embodiments of the present invention. In some embodiments, display 40 may comprise a touchscreen that can be configured to accept inputs from operator 32, in addition to presenting image 38.

Based on the signals received from medical probe 22 and other components of medical system 20, control console 26 drives display 40 to update image 38 to present a current position of distal end 34 in the patient's body, as well as status information and guidance regarding the procedure that is in progress. In some embodiments, operator 32 can manipulate image 38 using one or more input devices 42. In embodiments, where display 40 comprises a touchscreen display, operator 32 can manipulate image 38 via the touchscreen display.

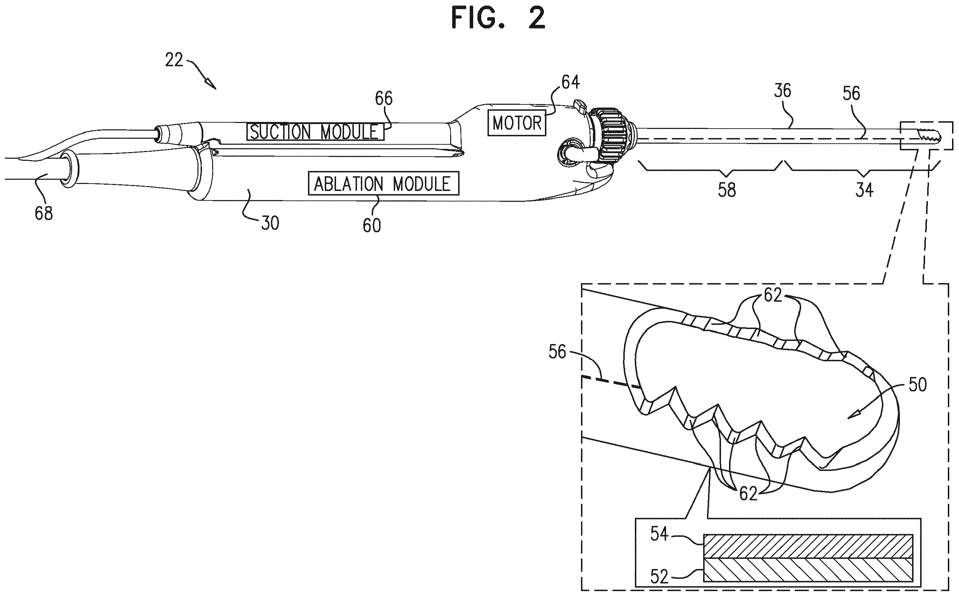

Figure 2 is a schematic sectional view of medical probe 22, in accordance with an embodiment of the present invention. Outer tube 36 comprises a radial cutting window opening 50 in proximity to distal end 34. In embodiments of the present invention, probe 22 comprises a conductive element 52 that is disposed over the distal end of insertion tube 35. In some embodiments, conductive element 52 is disposed over an outer surface 54 of outer tube 36. Outer surface 54 typically comprises an isolating polymer such as Teflon^{™} or polyurethane, and conductive element 52 typically comprises a thin layer of metal (e.g., gold) that is configured to inject energy into patient 28 in order to perform coagulation.

Medical probe 22 also comprises a conductor 56, typically in the form of one or more conducting wires. In embodiments where conductive element 52 is disposed over outer tube 36, conductor 56 runs along the outer tube from a proximal end 58 of the outer tube to distal end 34. Handle 30 comprises a coagulation module 60 that is coupled to conductor 56 at proximal end 58, and the conductor is coupled to conductive element 52 at distal end 34. Coagulation module 60 conveys energy (e.g., radio-frequency energy) to conductive element 52, and is configured to monitor and control coagulation parameters such as the level and the duration of the energy conveyed to the conductive element. Conductive element 52 injects the conveyed energy into a body cavity (e.g., a nasal cavity and/or a paranasal sinus) of patient 28 in order to generate heat that can be used to coagulate fluid (e.g., blood) in the body cavity.

Distal end 34 comprises an outer blade 62 disposed along one or more edges of radial cutting window opening 50. In the configuration shown in Figure 2, outer blade 62 may comprise a set of grasping teeth that can be used to grasp tissue in a body cavity during a medical procedure. In some embodiments, outer blade 62 may be coated with conductive element 52, thereby enabling the grasping teeth to perform coagulation during the medical procedure.

Handle 30 also comprises a motor 64 and a suction module 66. Details and operation of motor 64 and suction module 66 are described hereinbelow in the description referencing Figures 3 and 4. While the configuration in Figure 2 shows handle components (i.e., coagulation module 60, motor 64 and suction module 66) in handle 30 receiving electrical energy from a cable 68, powering the handle components from any other electrical energy source is considered to be within the scope of the present invention. For example, the handle may comprise a battery that conveys the electrical energy to the modules in the handle.

Figures 3 and 4 are schematic sectional views of distal end 34 comprising inner tube 70 contained within outer tube 36, in accordance with an embodiment of the present invention. A proximal end of inner tube 70 is coupled to motor 64 that is configured to rotate the inner tube within outer tube 36 as indicated by an arrow 72, and a distal end of inner tube 70 comprises a cutting blade 74 that traverses radial cutting window opening 50 as motor 64 rotates the inner tube within outer tube 36, as shown in Figures 3 and 4. In operation, any debris produced as a result of coagulation (i.e., as a result of heat generated by conductive element 52) or cutting (i.e., by cutting blade 74) can be extracted upon the debris being produced.

In the configuration shown in Figure 3, the distal end of inner tube 70 comprises a radial suction window opening 76 and cutting blade 74 is disposed along an edge of the radial suction window opening. In operation, as motor 64 rotates inner tube 70, radial suction window opening 76 traverses radial cutting window opening 50 at specific angles of rotation, as motor 64 rotates the inner tube within outer tube 36. Additionally, distal end 34 can be coupled to suction module 66 so that debris (e.g., tissue and/or coagulated blood) can be extracted from a body cavity when radial suction window opening 76 is aligned with radial cutting window opening 50, as shown in Figure 3.

In some embodiments, conductive element 52 is disposed over an outer surface (not shown) of inner tube 70. In these embodiments, conductor 56 can run from a proximal end of inner tube 70 to distal end 34, and medical system 20 can be configured to restrict conveyance of energy to conductive element 52 to times when radial suction window opening 76 is opposite radial cutting window opening 50, i.e., when rotation of cutting blade 74 closes radial cutting window 50, as shown in Figure 4. In operation, as motor 64 rotates inner tube 70, coagulation module 60 can pulse, e.g., when cutting blade 74 closes radial cutting window 50, the energy conveyed to the conductive element disposed over the inner tube.

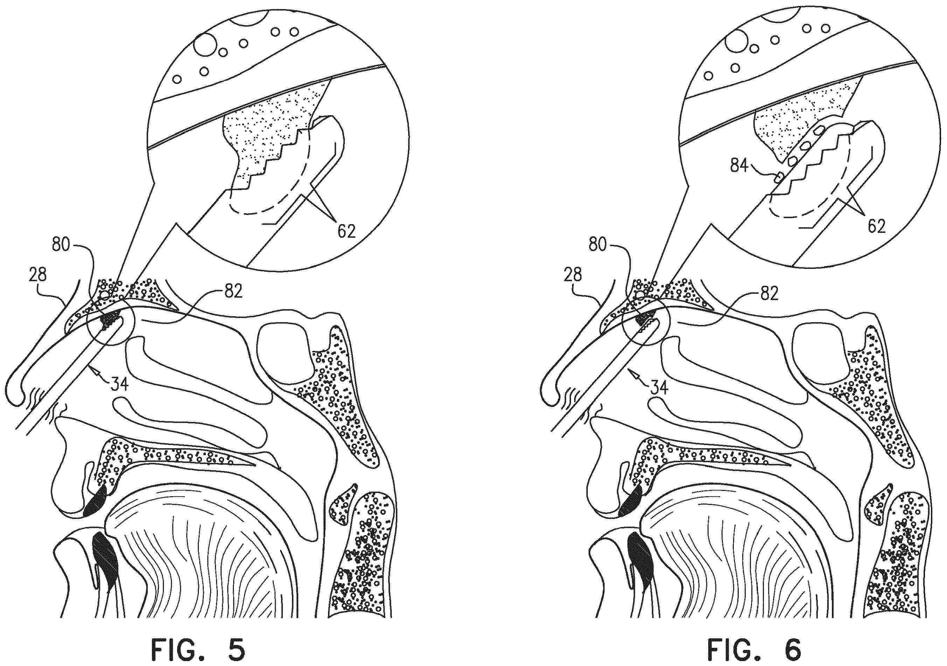

Figures 5 and 6 are schematic detail views showing distal end 34 in contact with sinus tissue 80 in a sinus cavity 82 during an exemplary medical procedure. In Figure 5, operator 32 positions distal end 34 in sinus cavity 82 so that the grasping teeth of outer blade 62 grasp sinus tissue 80. During the medical procedure cutting blade 74 removes some of the sinus tissue, as shown in Figure 6. Additionally, heat generated by conductive element 52 can coagulate blood or fluid in sinus cavity 82, and any debris 84 (e.g., blood coagulated by the conductive element and/or sinus tissue cut by cutting blade 74) can be removed from the sinus cavity via radial suction window opening 76.

Operator 32 can use input devices 42 (or any other type of input devices such as pedal switches or buttons on handle 30) to separately control coagulation module 60, motor 64 and suction module 66. This enables operator 32 to control the flow of energy from coagulation module 60 to conductive element 52 in order to vary the times of coagulation, and to control the flow of energy to motor 64 in order vary the cutting times and the speed of cutting blade 74 (i.e., by controlling the rotation of inner tube 70).

In additional embodiments, medical system 20 can be configured to independently control the rotation of the inner tube and the energy conveyed to the conductive element. For example, control console 26 may comprise a first given input device 42 that operator 32 can manipulate in order to control motor 64 and a second given input device 42 that operator 32 can manipulate in order to control coagulation module 60. In alternative embodiments, medical system 20 may comprise other types of input devices (e.g., pedal switches) that operator 32 can manipulate in order to independently control motor 64 and coagulation module 60. By enabling operator 32 to independently control motor 64 and coagulation module 60, medical system 20 enables operator 32 to vary the ratio of debriding (i.e., cutting tissue using cutting blade 74) and coagulation (i.e., using conductive element 52 to heat blood in sinus cavity 82) times.

It will be appreciated that the embodiments described above are cited by way of example, and that the present invention is not limited to what has been particularly shown and described hereinabove. Rather, the scope of the present invention is defined solely by the appended claims.

## Claims

1. A medical apparatus (22), comprising:
an insertion tube (35) having a distal end (34) and comprising:
an outer tube (36) having a proximal end and an outer tube distal end configured for insertion into a body cavity, with a radial cutting window opening (50) through a wall of the outer tube in proximity to the distal end, and
an inner tube (70), contained in and configured to rotate within the outer tube and comprising a cutting blade (74) that traverses the radial cutting window opening as the inner tube rotates;
a conductive element (52) disposed over an outer surface of the outer tube distal end, wherein the conductive element is configured to inject energy into the body cavity in order to perform coagulation; and
a conductor (56) running along the insertion tube from a proximal to the distal end of the insertion tube and coupled to provide energy to the conductive element,
wherein the inner tube has an inner tube distal end and an inner tube proximal end, and comprising a radial suction window opening (76) through a wall of the inner tube in proximity to the inner tube distal end, the radial suction window opening being configured to traverse the radial cutting window opening at specific angles of rotation, and wherein the inner tube proximal end is coupled to a suction device configured to extract debris via the radial suction window opening,
**characterised in that** the medical apparatus is configured to restrict conveyance of energy to the conductive elements to times when the radial cutting window opening and the radial suction window opening are aligned.

2. The medical apparatus according to claim 1, wherein the cutting blade (74) comprises a first cutting blade (74), and comprising a second cutting blade (62) disposed on an edge of the radial cutting window opening (50).

3. The medical apparatus according to claim 1, wherein the cutting blade (74) is disposed on an edge of the radial suction window opening (76).

4. The medical apparatus according to claim 1, and comprising input devices (60, 64) that independently control the rotation of the inner tube (70) and the provision of the energy to the conductive element (52) in order to vary a ratio of cutting and heating times.

5. A method of providing a medical apparatus, comprising:
providing an insertion tube (35) configured for insertion into a body cavity and comprising an outer tube (36) having a proximal end and a distal end and an inner tube (70) contained in and configured to rotate within the outer tube;
configuring, in the outer tube, a radial cutting window opening (50) through a wall of the outer tube in proximity to the distal end;
positioning, in the inner tube, a cutting blade (74) that traverses the radial cutting window as the inner tube rotates;
providing a conductive element (52), configured to inject energy into the body cavity in order to perform coagulation, disposed over an outer surface of the distal end of the outer tube;
providing a conductor (56) running along the insertion tube from the proximal to the distal end and coupled to provide energy to the conductive element;
wherein the distal end comprises an outer tube distal end, wherein the inner tube has an inner tube distal end and an inner tube proximal end, and the method comprising providing a radial suction window opening (76) through a wall of the inner tube in proximity to the inner tube distal end, the radial suction window opening being configured to traverse the radial cutting window opening at specific angles of rotation, and providing, at the inner tube proximal end, a coupling for a suction device configured to extract debris via the radial suction window opening; and
configuring the medical apparatus to restrict conveyance of energy to the conductive elements to times when the radial cutting window opening and the radial suction window openings are aligned.

6. The method according to claim 5, wherein the cutting blade (74) comprises a first cutting blade (74), and comprising disposing a second cutting blade (62) on an edge of the radial cutting window opening (50).

7. The medical apparatus according to claim 2 or the method according to claim 6, wherein the second cutting blade (62) comprises a set of grasping teeth configured to grasp body cavity tissue.

8. The medical apparatus according to claim 2, wherein the conductive element (52) is disposed over the second cutting blade (62).

9. The method according to claim 6, and comprising disposing the conductive element (52) over the second cutting blade (62).

10. The method according to claim 5, and comprising disposing the cutting blade (74) on an edge of the radial suction window opening (76).

11. The medical apparatus according to claim 1 or the method according to claim 5, wherein the body cavity is selected from a group consisting of a nasal cavity and a paranasal sinus.

12. The medical apparatus according to claim 1 or the method according to claim 5, wherein the energy comprises radio-frequency energy.

## Patentansprüche

1. Medizinisches Gerät (22), umfassend:
ein Einführrohr (35) mit einem distalen Ende (34), und umfassend:
ein Außenrohr (36) mit einem proximalen Ende und einem distalen Ende des Außenrohrs, das zum Einführen in eine Körperhöhle ausgestaltet ist, mit einer radialen Schneidfensteröffnung (50) durch eine Wand des Außenrohrs hindurch in der Nähe des distalen Endes, und
ein Innenrohr (70), das in dem Außenrohr enthalten ist und ausgestaltet ist, um in diesem zu rotieren, und umfassend eine Schneidklinge (74), welche die radiale Schneidfensteröffnung durchquert, wenn das Innenrohr rotiert;
ein leitfähiges Element (52), das über einer Außenfläche des distalen Endes des Außenrohrs angeordnet ist, wobei das leitfähige Element ausgestaltet ist, um Energie in die Körperhöhle einzuspeisen, um Koagulation durchzuführen; und
einen Leiter (56), der entlang des Einführrohrs von einem proximalen zu dem distalen Ende des Einführrohrs verläuft und gekoppelt ist, um dem leitfähigen Element Energie bereitzustellen,
wobei das Innenrohr ein distales Ende des Innenrohrs und ein proximales Ende des Innenrohrs hat, und umfassend eine radiale Saugfensteröffnung (76) durch eine Wand des Innenrohrs hindurch in der Nähe des distalen Endes des Innenrohrs, wobei die radiale Saugfensteröffnung ausgestaltet ist, um die radiale Schneidfensteröffnung bei speziellen Rotationswinkeln zu queren, und wobei das proximale Ende des Innenrohrs an eine Saugvorrichtung gekoppelt ist, die ausgestaltet ist, um Trümmer über die radiale Saugfensteröffnung zu extrahieren,
**dadurch gekennzeichnet, dass** das medizinische Gerät ausgestaltet ist, um Transport von Energie zu den leitfähigen Elementen auf Zeiten zu begrenzen, wenn die radiale Schneidfensteröffnung und die radiale Saugfensteröffnung ausgerichtet sind.

2. Medizinisches Gerät nach Anspruch 1, wobei die Schneidklinge (74) eine erste Schneidklinge (74) umfasst, und umfassend eine zweite Schneidklinge (62), die auf einem Rand der radialen Schneidfensteröffnung (50) angeordnet ist.

3. Medizinisches Gerät nach Anspruch 1, wobei die Schneidklinge (74) auf einem Rand der radialen Saugfensteröffnung (76) angeordnet ist.

4. Medizinisches Gerät nach Anspruch 1, und umfassend Eingabevorrichtungen (60, 64), die unabhängig die Rotation des Innenrohrs (70) und die Bereitstellung der Energie an das leitfähige Element (52) steuern, um ein Verhältnis von Schneid- und Heizzeiten zu variieren.

5. Verfahren zum Bereitstellen eines medizinischen Geräts, umfassend:
Bereitstellen eines Einführrohrs (35), das zum Einführen in eine Körperhöhle ausgestaltet ist und ein Außenrohr (36) mit einem proximalen Ende und einem distalen Ende und ein Innenrohr (70) umfasst, das in dem Außenrohr enthalten ist und ausgestaltet ist, um in diesem zu rotieren;
Ausgestalten einer radialen Schneidfensteröffnung (50) durch eine Wand des Außenrohrs hindurch in der Nähe des distalen Endes in dem Außenrohr;
Positionieren einer Schneidklinge (74), welche das radiale Schneidfenster quert, wenn das Innenrohr rotiert, in dem Innenrohr;
Bereitstellen eines leitfähigen Elements (52), das ausgestaltet ist, um Energie in die Körperhöhle einzuspeisen, um Koagulation durchzuführen, und über einer Außenfläche des distalen Endes des Außenrohrs angeordnet ist;
Bereitstellen eines Leiters (56), der entlang des Einführrohrs von dem proximalen zu dem distalen Ende verläuft und gekoppelt ist, um dem leitfähigen Element Energie bereitzustellen;
wobei das distale Ende ein distales Ende des Außenrohrs umfasst, wobei das Innenrohr ein distales Ende des Innenrohrs und ein proximales Ende des Innenrohrs hat, und das Verfahren Bereitstellen einer radialen Saugfensteröffnung (76) durch eine Wand des Innenrohrs hindurch in der Nähe des distalen Endes des Innenrohrs umfasst, wobei die radiale Saugfensteröffnung ausgestaltet ist, um die radiale Schneidfensteröffnung bei speziellen Rotationswinkeln zu queren, und Bereitstellen einer Kopplung für eine Saugvorrichtung an dem proximalen Ende des Innenrohrs, wobei die Saugvorrichtung ausgestaltet ist, um Trümmer über die radiale Saugfensteröffnung zu extrahieren; und
Ausgestalten des medizinischen Geräts, um Transport von Energie zu den leitfähigen Elementen auf Zeiten zu begrenzen, wenn die radiale Schneidfensteröffnung und die radialen Saugfensteröffnungen ausgerichtet sind.

6. Verfahren nach Anspruch 5, wobei die Schneidklinge (74) eine erste Schneidklinge (74) umfasst, und umfassend Anordnen einer zweiten Schneidklinge (62) auf einem Rand der radialen Schneidfensteröffnung (50) .

7. Medizinisches Gerät nach Anspruch 2 oder Verfahren nach Anspruch 6, wobei die zweite Schneidklinge (62) einen Satz von Greifzähnen umfasst, die ausgestaltet sind, um Gewebe der Körperhöhle zu greifen.

8. Medizinisches Gerät nach Anspruch 2, wobei das leitfähige Element (52) über der zweiten Schneidklinge (62) angeordnet ist.

9. Verfahren nach Anspruch 6, und umfassend Anordnen des leitfähigen Elements (52) über der zweiten Schneidklinge (62).

10. Verfahren nach Anspruch 5, und umfassend Anordnen der Schneidklinge (74) auf einem Rand der radialen Saugfensteröffnung (76).

11. Medizinisches Gerät nach Anspruch 1 oder Verfahren nach Anspruch 5, wobei die Körperhöhle ausgewählt ist aus einer Gruppe bestehend aus einer Nasenhöhle und Nasennebenhöhle.

12. Medizinisches Gerät nach Anspruch 1 oder Verfahren nach Anspruch 5, wobei die Energie Hochfrequenzenergie umfasst.

## Revendications

1. Appareil médical (22), comprenant :
un tube d'insertion (35) ayant une extrémité distale (34) et comprenant :
un tube externe (36) ayant une extrémité proximale et une extrémité distale de tube externe configurée pour l'insertion dans une cavité corporelle, avec une ouverture de fenêtre de coupe radiale (50) à travers une paroi du tube externe à proximité de l'extrémité distale, et
un tube interne (70), contenu dans et configuré pour tourner à l'intérieur du tube externe et comprenant une lame de coupe (74) qui traverse l'ouverture de fenêtre de coupe radiale lorsque le tube interne tourne ;
un élément conducteur (52) disposé sur une surface extérieure de l'extrémité distale du tube externe, l'élément conducteur étant configuré pour injecter de l'énergie dans la cavité corporelle afin de réaliser une coagulation ; et
un conducteur (56) s'étendant le long du tube d'insertion depuis une extrémité proximale jusqu'à l'extrémité distale du tube d'insertion et couplé pour fournir de l'énergie à l'élément conducteur,
le tube interne ayant une extrémité distale de tube interne et une extrémité proximale de tube interne, et comprenant une ouverture de fenêtre d'aspiration radiale (76) à travers une paroi du tube interne à proximité de l'extrémité distale du tube interne, l'ouverture de fenêtre d'aspiration radiale étant configurée pour traverser l'ouverture de fenêtre de coupe radiale à des angles de rotation spécifiques, et l'extrémité proximale du tube interne étant couplée à un dispositif d'aspiration configuré pour extraire des débris via l'ouverture de fenêtre d'aspiration radiale,
**caractérisé en ce que** l'appareil médical est configuré pour restreindre le transport d'énergie vers les éléments conducteurs aux moments où l'ouverture de fenêtre de coupe radiale et l'ouverture de fenêtre d'aspiration radiale sont alignées.

2. Appareil médical selon la revendication 1, la lame de coupe (74) comprenant une première lame de coupe (74), et comprenant une seconde lame de coupe (62) disposée sur un bord de l'ouverture de fenêtre de coupe radiale (50).

3. Appareil médical selon la revendication 1, la lame de coupe (74) étant disposée sur un bord de l'ouverture de fenêtre d'aspiration radiale (76).

4. Appareil médical selon la revendication 1, et comprenant des dispositifs d'entrée (60, 64) qui commandent indépendamment la rotation du tube interne (70) et la fourniture de l'énergie à l'élément conducteur (52) afin de faire varier un rapport entre les temps de coupe et de chauffage.

5. Procédé de fourniture d'un appareil médical, comprenant :
la fourniture d'un tube d'insertion (35) configuré pour être inséré dans une cavité corporelle et comprenant un tube externe (36) ayant une extrémité proximale et une extrémité distale, et un tube interne (70) contenu dans et configuré pour tourner à l'intérieur du tube externe ;
la configuration, dans le tube externe, d'une ouverture de fenêtre de coupe radiale (50) à travers une paroi du tube externe à proximité de l'extrémité distale ;
le positionnement, dans le tube interne, d'une lame de coupe (74) qui traverse la fenêtre de coupe radiale lorsque le tube interne tourne ;
la fourniture d'un élément conducteur (52), configuré pour injecter de l'énergie dans la cavité corporelle afin de réaliser une coagulation, disposé sur une surface extérieure de l'extrémité distale du tube externe ;
la fourniture d'un conducteur (56) s'étendant le long du tube d'insertion de l'extrémité proximale jusqu'à l'extrémité distale, et couplé pour fournir de l'énergie à l'élément conducteur ;
l'extrémité distale comprenant une extrémité distale de tube externe, le tube interne ayant une extrémité distale de tube interne et une extrémité proximale de tube interne, et le procédé comprenant la fourniture d'une ouverture de fenêtre d'aspiration radiale (76) à travers une paroi du tube interne à proximité de l'extrémité distale du tube interne, l'ouverture de fenêtre d'aspiration radiale étant configurée pour traverser l'ouverture de fenêtre de coupe radiale à des angles de rotation spécifiques, et la fourniture, à l'extrémité proximale du tube interne, d'un couplage pour un dispositif d'aspiration configuré pour extraire des débris via l'ouverture de fenêtre d'aspiration radiale ; et
la configuration de l'appareil médical pour restreindre le transport d'énergie vers les éléments conducteurs aux moments où l'ouverture de fenêtre de coupe radiale et l'ouverture de fenêtre d'aspiration radiale sont alignées.

6. Procédé selon la revendication 5, la lame de coupe (74) comprenant une première lame de coupe (74), et comprenant la disposition d'une seconde lame de coupe (62) sur un bord de l'ouverture de fenêtre de coupe radiale (50).

7. Appareil médical selon la revendication 2 ou procédé selon la revendication 6, la seconde lame de coupe (62) comprenant un ensemble de dents de préhension configurées pour saisir le tissu de la cavité corporelle.

8. Appareil médical selon la revendication 2, l'élément conducteur (52) étant disposé au-dessus de la seconde lame de coupe (62).

9. Procédé selon la revendication 6, et comprenant la disposition de l'élément conducteur (52) sur la seconde lame de coupe (62).

10. Procédé selon la revendication 5, et comprenant la disposition de la lame de coupe (74) sur un bord de l'ouverture de fenêtre d'aspiration radiale (76).

11. Appareil médical selon la revendication 1 ou procédé selon la revendication 5, la cavité corporelle étant choisie dans un groupe constitué par une cavité nasale et un sinus paranasal.

12. Appareil médical selon la revendication 1 ou procédé selon la revendication 5, l'énergie comprenant une énergie radiofréquence.
